# EUROPEAN PATENT SPECIFICATION

(11) **EP 0 857 173 B1**
(45) Date of publication and mention of the grant of the patent: **19.11.2003**
(21) Application number: 96918629.5
(22) Date of filing: 21.06.1996
(51) Int. Cl.: C07J 9/00, A61K 31/575

(54) **MEIOSIS REGULATING COMPOUNDS**
MEIOSE REGULIERENDE VERBINDUNGEN.
COMPOSES REGULATEURS DE LA MEIOSE

(30) Priority: 23.06.1995 DK 72895; 23.06.1995 DK 73095; 22.12.1995 DK 146195
(43) Date of publication of application: 12.08.1998
(73) Proprietor: NOVO NORDISK A/S, 2880 Bagsvaerd (DK)
(72) Inventor: GROENVALD, Frederik, Christian, DK-2950 Vedbaek (DK); FAARUP, Peter, DK-3500 Vaerloese (DK); GUDDAL, Erling, DK-2605 Broendby (DK)
(86) International application number: DK9600273
(87) International publication number: WO97000884

(56) References cited:
- WO-A-96/00235
- INTERNATIONAL, File CAPLUS, CAPLUS accession no. 1991:472013, FRELEK, JADWIGA et al., "Chiroptical Properties of Stereoisomeric Conjugated Oximes"; & TETRAHEDRON: ASYMMETRY, 2(5), 381-7 (ENGLISH) 1991, see CASRN 135129-00-9.
- STEROIDS, Volume 48, 1986, EDWARD J. PARISH et al., "Synthesis of 3 beta-Hydroxy-5 alpha-Cholest-8-En-7-One and 3 beta-Hydroxy-5 alpha-Cholest-8-En-11-One: Evaluation as Potential Hypocholesterolemic Agents", page 407 - page 418.
- INTERNATIONAL, File CAPLUS, CAPLUS accession no. 1983:422756, ANASTASIA, MARIO et al., "A New Route to Steroid Ring-C Aromatization from 7-Oxygenated Steroids"; & J. CHEM. SOC., PERKIN TRANS. 1(3), 587-90 (ENGLISH) 1983, see CASRN 69140-15-4, 63115-68-4.
- INTERNATIONAL, File CAPLUS, CAPLUS accession no. 1989:566883, PARISH, EDWARD J. et al., "Studies of the Oxysterol Inhibition of Tumor Cell Growth"; & STEROIDS, 53(3-5), 579-96 (ENGLISH) 1989, see CASRN 62250-89-9.
- INTERNATIONAL, File CAPLUS, CAPLUS accession no. 1979:420894, PATTERSON, DONALD G. et al., "Stereochemical Course of the Chemical and Catalytic Reduction of 11-Oxo-5.alpha., 14 beta.-Cholest-8-En-3.beta.-Ol. Synthesis of 8.alpha., 9.alpha.,14 beta.-,8.alpha-.,9 beta., 14 beta.-, and 8 beta., 9 alpha., 14.beta.-Steroids"; & J. ORG. CHEM., 44(11), 1866-71 (ENGLISH) 1979, see CASRN 69454-76-8, 69454-77-9, 62279-64-5.

## Description

### FIELD OF THE INVENTION

The present invention relates to the use of certain pharmacologically active for regulating the meiosis.

### BACKGROUND OF THE INVENTION

Meiosis is the unique and ultimate event of germ cells on which sexual reproduction is based. Meiosis comprises two meiotic divisions. During the first division, exchange between maternal and paternal genes take place before the pairs of chromosomes are separated into the two daughter cells. These contain only half the number (1 n) of chromosomes and 2c DNA. The second meiotic division proceeds without a DNA synthesis. This division therefore results in the formation of the haploid germ cells with only 1 c DNA.

The meiotic events are similar in the male and female germ cells, but the time schedule and the differentiation processes which lead to ova and to spermatozoa differ profoundly. All female germ cells enter the prophase of the first meiotic division early in life, often before birth, but all are arrested as oocytes later in the prophase (dictyate state) until ovulation after puberty. Thus, from early life the female has a stock of oocytes which is drawn upon until the stock is exhausted. Meiosis in females is not completed until after fertilization, and results in only one ovum and two abortive polar bodies per germ cell. In contrast, only some of the male germ cells enter meiosis from puberty and leave a stem population of germ cells throughout life. Once initiated, meiosis in the male cell proceeds without significant delay and produces 4 spermatozoa.

Only little is known about the mechanisms which control the initiation of meiosis in the male and in the female. In the oocyte, new studies indicate that follicular purines, hypoxanthine or adenosine, could be responsible for meiotic arrest (Downs, SM *et al. Dev Biol* **82** (1985) 454-458; Eppig, JJ *et al. Dev Biol* **119** (1986) 313-321; and Downs, SM *Mol Reprod Dev* **35** (1993) 82-94). The presence of a diffusible meiosis regulating substance was first described by Byskov et al. in a culture system of fetal mouse gonads (Byskov, AG *et al. Dev Biol* **52** (1976) 193-200). A meiosis activating substance (MAS) was secreted by the fetal mouse ovary in which meiosis was ongoing, and a meiosis preventing substance (MPS) was released from the morphologically differentiated testis with resting, non-meiotic germ cells. It was suggested that the relative concentrations of MAS and MPS regulated the beginning, arrest and resumption of meiosis in the male and in the female germ cells (Byskov, AG *et al.* in *The Physiology of Reproduction* (eds. Knobil, E and Neill, JD, Raven Press, New York (1994)). Clearly, if meiosis can be regulated, reproduction can be controlled. A recent article (Byskov, AG *et al. Nature* **374** (1995) 559-562) describes the isolation from bull testes and from human follicular fluid of certain sterols that activate oocyte meiosis. Unfortunately, these sterols are rather labile and utilization of the interesting finding would thus be greatly facilitated if more stable meiosis activating compounds were available.

In a paper with the title "Synthesis of 3β-hydroxy-5α-cholest-8-en-7-one and 3β-hydroxy-5α-cholest-8-en-11-one: Evaluation as potential hypocholesterolemic agents" in *Steroids* 48 (1986), 407-418, *inter alia* the following compounds are mentioned: 3β-hydroxy-5α-cholest-8-en-7-one, 3β-hydroxy-5α-cholest-8-en-11-one, 7-oxo-5α-cholest-8-en-3β-yl benzoate, and 11-oxo-5α-cholest-8-en-3β-yl benzoate.

### SUMMARY OF THE INVENTION

It is a purpose of the present invention to provide the use of certain compounds and methods useful for relieving infertility in females and males, particularly in mammals, more particularly in humans.

It is a further purpose of the present invention to provide the use of certain compounds and methods useful as contraceptives in females and males, particularly in mammals, more particularly in humans.

The compounds mentioned herein can be used for regulating the meiosis in oocytes and in male germ cells.

In one aspect, the present invention relates to the use of compounds of the general formula (I) 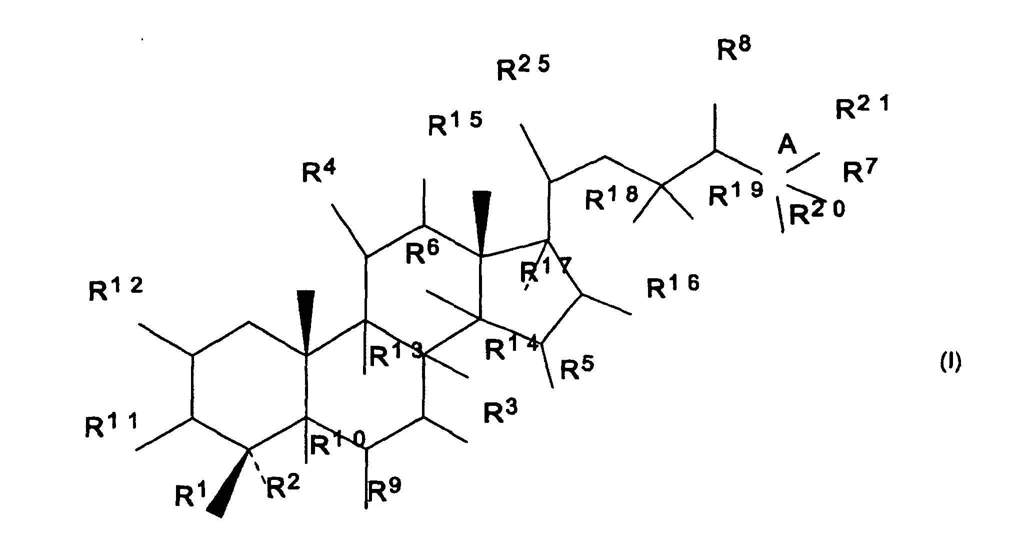 wherein R¹ and R², independently, are selected from the group comprising hydrogen and branched or unbranched C₁-C₆ alkyl which may be substituted by halogen, hydroxy or cyano, or wherein R¹ and R² together designate methylene or, together with the carbon atom to which they are bound, form a cyclopropane ring, a cyclopentane ring, or a cyclohexane ring; R³ is selected from the group comprising hydrogen, methylene, hydroxy, methoxy, acetoxy, oxo, =NOR²⁶ wherein R²⁶ is hydrogen or C₁-C₃ alkyl, halogen, and hydroxy and C₁-C₄ alkyl bound to the same carbon atom of the sterol skeleton, or R³ designates, together with R⁹ or R¹⁴, an additional bond between the carbon atoms to which R³ and R⁹ or R¹⁴ are bound; R⁴ is selected from the group comprising hydrogen, methylene, hydroxy, methoxy, acetoxy, oxo, =NOR²⁷ wherein R²⁷ is hydrogen or C₁-C₃ alkyl, halogen, and hydroxy and C₁-C₄ alkyl bound to the same carbon atom of the sterol skeleton, or R⁴ designates, together with R¹³ or R¹⁵, an additional bond between the carbon atoms to which R⁴ and R¹³ or R¹⁵ are bound; R⁵ is selected from the group comprising hydrogen, C₁-C₄ alkyl, methylene, hydroxy, methoxy, oxo, and =NOR²² wherein R²² is hydrogen or C₁-C₃ alkyl, or R⁵ designates, together with R⁶, an additional bond between the carbon atoms to which R⁵ and R⁶ are bound; R⁶ is hydrogen or R⁶ designates, together with R⁵, an additional bond between the carbon atoms to which R⁵ and R⁶ are bound; R⁹ is hydrogen or R⁹ designates, together with R³ or R¹⁰, an additional bond between the carbon atoms to which R⁹ and R³ or R¹⁰ are bound; R¹⁰ is hydrogen or R¹⁰ designates, together with R⁹, an additional bond between the carbon atoms to which R¹⁰ and R⁹ are bound; R¹¹ is selected from the group comprising hydroxy, alkoxy, substituted alkoxy, acyloxy, sulphonyloxy, phosphonyloxy, oxo, =NOR²⁸ wherein R²⁸ is hydrogen or C₁ -C₃ alkyl, halogen and hydroxy and C₁-C₄ alkyl bound to the same carbon atom of the sterol skeleton, or R¹¹ designates, together with R¹², an additional bond between the carbon atoms to which R¹¹ and R¹² are bound; R¹² is selected from the group comprising hydrogen, C₁-C₃ alkyl, vinyl, C₁-C₃ alkoxy and halogen, or R¹² designates, together with R ¹¹, an additional bond between the carbon atoms to which R¹² and R¹¹ are bound; R¹³ is hydrogen or R¹³ designates, together with R⁴ or R¹⁴, an additional bond between the carbon atoms to which R¹³ and R⁴ or R¹⁴ are bound; R¹⁴ is hydrogen or R¹⁴ designates, together with R³, R⁶ or R¹³, an additional bond between the carbon atoms to which R¹⁴ and R³ or R⁶ or R¹³ are bound; R¹⁵ is selected from the group comprising hydrogen, C₁-C ₄ alkyl, methylene, hydroxy, methoxy, acetoxy, oxo, and =NOR²³ wherein R²³ is hydrogen or C₁-C₃ alkyl, or R¹⁵ designates, together with R⁴, an additional bond between the carbon atoms to which R¹⁵ and R⁴ are bound; R¹⁶ is selected from the group comprising hydrogen, C₁-C₃ alkyl, methylene, hydroxy, methoxy, oxo and =NOR²⁴ wherein R²⁴ is hydrogen or C₁-C₃ alkyl, or R¹⁶ designates, together with R¹⁷, an additional bond between the carbon atoms to which R¹⁶ and R¹⁷ are bound; R¹⁷ is hydrogen or R¹⁷ designates, together with R¹⁶, an additional bond between the carbon atoms to which R¹⁷ and R¹⁶ are bound; R¹⁸ and R¹⁹ are independently hydrogen or fluoro; R²⁵ is selected from the group comprising C₁₋₄ alkyl, methylene, hydroxy and oxo; A is a carbon atom or a nitrogen atom; when A is a carbon atom, R⁷ is selected from the group comprising hydrogen, hydroxy and fluoro, and R⁸ is selected from the group comprising hydrogen, C₁-C₄ alkyl, methylene and halogen, or R⁷ designates, together with R⁸, an additional bond between the carbon atoms to which R⁷ and R⁸ are bound; R²⁰ is selected from the group comprising C₁-C₄ alkyl, trifluoromethyl and C₃-C₆ cycloalkyl and R²¹ is selected from the group comprising C₁-C₄ alkyl, C₁-C₄ hydroxyalkyl, C₁-C₄ haloalkyl containing up to three halogen atoms, methoxymethyl, acetoxymethyl, and C₃-C₆ cycloalkyl, or R²⁰ and R²¹, together with the carbon atom to which they are bound, form a C₃-C₆ cydoalkyl ring; and when A is a nitrogen atom, R⁷ designates a lone pair of electrons and R⁸ is selected from the group comprising hydrogen, C₁-C₄ alkyl and oxo; R²⁰ and R²¹ are, independently, C₁-C₄ alkyl or C₃-C₆ cycloalkyl; with the proviso that the compound of the general formula (I) does not have any cumulated double bonds and with the further proviso that the compound is not a compound of the general formula (II) 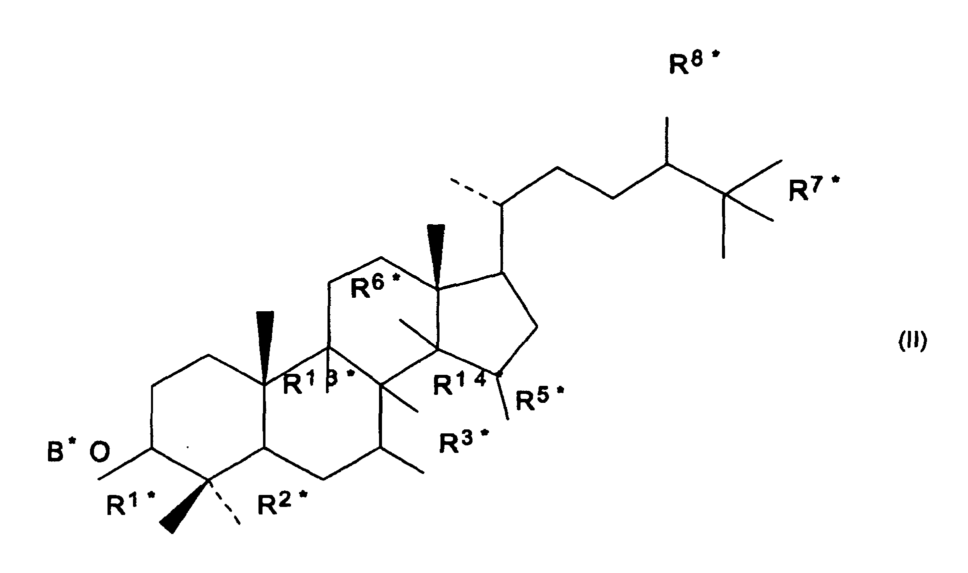 wherein R^{1*} and R^{2*}, independently, are selected from the group comprising hydrogen, branched or unbranched C₁-C₆ alkyl which may be substituted by halogen or hydroxy or wherein R^{1*} and R^{2*}, together with the carbon atom to which they are bound, form a cyclopentane ring or a cyclohexane ring; R^{13*} and R^{14*} together designate an additional bond between the carbon atoms to which they are bound in which case R^{3*} is hydrogen and R^{6*} and R^{5*} are either hydrogen or together they designate an additional bond between the carbon atoms to which they are bound; or R^{3*} and R^{14*} together designate an additional bond between the carbon atoms to which they are bound in which case R^{13*} is hydrogen and R^{6*} and R^{5*} are either hydrogen or together they designate an additional bond between the carbon atoms to which they are bound; or R^{6*} and R^{14*} together designate an additional bond between the carbon atoms to which they are bound in which case R^{13*}, R^{3*} and R^{5*} are all hydrogen; R^{8*} and R^{7*} are hydrogen or together they designate an additional bond between the carbon atoms to which they are bound; and B* is either hydrogen or an acyl group, including a sulphonyl group or a phosphonyl group, or a group which together with the remaining part of the molecule forms an ether, and esters and ethers thereof for preparing a medicament to be used in the regulation of meiosis.

In a preferred embodiment, the compound of formula (I) above is a compound wherein R¹ and R² are both hydrogen.

In another preferred embodiment, the compound of formula (I) above is a compound wherein one of R¹ and R² is hydrogen while the other is methyl.

In another preferred embodiment, the compound of formula (I) above is a compound wherein R¹ and R² are both methyl.

In another preferred embodiment, the compound of formula (I) above is a compound wherein R¹ is branched or unbranched C₁-C₆ alkyl, optionally substituted by halogen, hydroxy or cyano.

In another preferred embodiment, the compound of formula (I) above is a compound wherein R² is branched or unbranched C₁-C₆ alkyl, optionally substituted by halogen, hydroxy or cyano.

In another preferred embodiment, the compound of formula (I) above is a compound wherein R¹ and R² together designate methylene.

In another preferred embodiment, the compound of formula (I) above is a compound wherein R¹ and R², together with the carbon atom to which they are bound, form a cyclopropane ring.

In another preferred embodiment, the compound of formula (I) above is a compound wherein R¹ and R², together with the carbon atom to which they are bound, form a cyclopentane ring.

In another preferred embodiment, the compound of formula (I) above is a compound wherein R¹ and R², together with the carbon atom to which they are bound, form a cyclohexane ring.

In another preferred embodiment, the compound of formula (I) above is a compound wherein R³ is hydrogen.

In another preferred embodiment, the compound of formula (I) above is a compound wherein R³ is methylene.

In another preferred embodiment, the compound of formula (I) above is a compound wherein R³ is hydroxy.

In another preferred embodiment, the compound of formula (I) above is a compound wherein R³ is methoxy or acetoxy.

In another preferred embodiment, the compound of formula (I) above is a compound wherein R³ is halogen.

In another preferred embodiment, the compound of formula (I) above is a compound wherein R³ is oxo.

In another preferred embodiment, the compound of formula (I) above is a compound wherein R³ is =NOH.

In another preferred embodiment, the compound of formula (I) above is a compound wherein R³ is =NOR²⁶, wherein R²⁶ is C₁-C₃ alkyl.

In another preferred embodiment, the compound of formula (I) above is a compound wherein R³ is hydroxy and C₁-C₄ alkyl bound to the same carbon atom of the sterol skeleton.

In another preferred embodiment, the compound of formula (I) above is a compound wherein R³, together with R⁹, designates an additional bond between the carbon atoms to which R³ and R⁹ are bound.

In another preferred embodiment, the compound of formula (I) above is a compound wherein R³, together with R¹⁴, designates an additional bond between the carbon atoms to which R³ and R¹⁴ are bound.

In another preferred embodiment, the compound of formula (I) above is a compound wherein R⁴ is hydrogen.

In another preferred embodiment, the compound of formula (I) above is a compound wherein R⁴ is methylene.

In another preferred embodiment, the compound of formula (I) above is a compound wherein R⁴ is hydroxy.

In another preferred embodiment, the compound of formula (I) above is a compound wherein R⁴ is methoxy or acetoxy.

In another preferred embodiment, the compound of formula (I) above is a compound wherein R⁴ is oxo.

In another preferred embodiment, the compound of formula (I) above is a compound wherein R⁴ is =NOH.

In another preferred embodiment, the compound of formula (I) above is a compound wherein R⁴ is =NOR²⁷, wherein R²⁷ is C₁-C₃ alkyl.

In another preferred embodiment, the compound of formula (I) above is a compound wherein R⁴ is hydroxy and C₁-C₄ alkyl bound to the same carbon atom of the sterol skeleton.

In another preferred embodiment, the compound of formula (I) above is a compound wherein R⁴, together with R¹³, designates an additional bond between the carbon atoms to which R⁴ and R¹³ are bound.

In another preferred embodiment, the compound of formula (I) above is a compound wherein R⁴, together with R¹⁵, designates an additional bond between the carbon atoms to which R⁴ and R¹⁵ are bound.

In another preferred embodiment, the compound of formula (I) above is a compound wherein R⁵ is hydrogen.

In another preferred embodiment, the compound of formula (I) above is a compound wherein R⁵ is C₁-C₄ alkyl.

In another preferred embodiment, the compound of formula (I) above is a compound wherein R⁵ is methylene.

In another preferred embodiment, the compound of formula (I) above is a compound wherein R⁵ is hydroxy.

In another preferred embodiment, the compound of formula (I) above is a compound wherein R⁵ is methoxy.

In another preferred embodiment, the compound of formula (I) above is a compound wherein R⁵ is oxo.

In another preferred embodiment, the compound of formula (I) above is a compound wherein R⁵ is =NOH.

In another preferred embodiment, the compound of formula (I) above is a compound wherein R⁵ is =NOR²², wherein R²² is C₁-C₃ alkyl.

In another preferred embodiment, the compound of formula (I) above is a compound wherein R⁵, together with R⁶, designates an additional bond between the carbon atoms to which R⁵ and R⁶ are bound.

In another preferred embodiment, the compound of formula (I) above is a compound wherein R⁶ is hydrogen.

In another preferred embodiment, the compound of formula (I) above is a compound wherein R⁶, together with R¹⁴, designates an additional bond between the carbon atoms to which R⁶ and R¹⁴ are bound.

In another preferred embodiment, the compound of formula (I) above is a compound wherein R⁹ is hydrogen.

In another preferred embodiment, the compound of formula (I) above is a compound wherein R⁹, together with R¹⁰, designates an additional bond between the carbon atoms to which R⁹ and R¹⁰ are bound.

In another preferred embodiment, the compound of formula (I) above is a compound wherein R¹⁰ is hydrogen.

In another preferred embodiment, the compound of formula (I) above is a compound wherein R¹¹ is hydroxy.

In another preferred embodiment, the compound of formula (I) above is a compound wherein R¹¹ is alkoxy, aralkyloxy, alkoxyalkoxy or alkanoyloxyalkyl, each group comprising a total of up to 10 carbon atoms, preferably up to 8 carbon atoms.

In another preferred embodiment, the compound of formula (I) above is a compound wherein R¹¹ is C₁-C₄ alkoxy.

In another preferred embodiment, the compound of formula (I) above is a compound wherein R¹¹ is methoxy.

In another preferred embodiment, the compound of formula (I) above is a compound wherein R¹¹ is ethoxy.

In another preferred embodiment, the compound of formula (I) above is a compound wherein R¹¹ is CH₃OCH₂O-.

In another preferred embodiment, the compound of formula (I) above is a compound wherein R¹¹ is pivaloyloxymethoxy.

In another preferred embodiment, the compound of formula (I) above is a compound wherein R¹¹ is an acyloxy group derived from an acid having from 1 to 20 carbon atoms.

In another preferred embodiment, the compound of formula (I) above is a compound wherein R¹¹ is an acyloxy group selected from the group comprising acetoxy, benzoyloxy, pivaloyloxy, butyryloxy, nicotinoyloxy, isonicotinoyloxy, hemi succinoyloxy, hemi glutaroyloxy, butylcarbamoyloxy, phenylcarbamoyloxy, butoxycarbonyloxy, tert-butoxycarbonyloxy and ethoxycarbonyloxy.

In another preferred embodiment, the compound of formula (I) above is a compound wherein R¹¹ is sulphonytoxy.

In another preferred embodiment, the compound of formula (I) above is a compound wherein R¹¹ is phosphonyloxy.

In another preferred embodiment, the compound of formula (I) above is a compound wherein R¹¹ is oxo.

In another preferred embodiment, the compound of formula (I) above is a compound wherein R¹¹ is =NOH.

In another preferred embodiment, the compound of formula (I) above is a compound wherein R¹¹ is =NOR²⁸, wherein R²⁸ is C₁-C₃ alkyl.

In another preferred embodiment, the compound of formula (I) above is a compound wherein R¹¹ is halogen.

In another preferred embodiment, the compound of formula (I) above is a compound wherein R¹¹ is hydroxy and C₁-C₄ alkyl bound to the same carbon atom of the sterol skeleton.

In another preferred embodiment, the compound of formula (I) above is a compound wherein R¹¹, together with R¹², designates an additional bond between the carbon atoms to which R¹¹ and R¹² are bound.

In another preferred embodiment, the compound of formula (I) above is a compound wherein R¹² is hydrogen.

In another preferred embodiment, the compound of formula (I) above is a compound wherein R¹² is C₁-C₃ alkyl.

In another preferred embodiment, the compound of formula (I) above is a compound wherein R¹² is C₁-C₃ alkoxy.

In another preferred embodiment, the compound of formula (I) above is a compound wherein R¹² is halogen.

In another preferred embodiment, the compound of formula (I) above is a compound wherein R¹³ is hydrogen.

In another preferred embodiment, the compound of formula (I) above is a compound wherein R¹³, together with R¹⁴, designates an additional bond between the carbon atoms to which R¹³ and R¹⁴ are bound.

In another preferred embodiment, the compound of formula (I) above is a compound wherein R¹⁴ is hydrogen.

In another preferred embodiment, the compound of formula (I) above is a compound wherein R¹⁵ is hydrogen.

In another preferred embodiment, the compound of formula (I) above is a compound wherein R¹⁵ is C₁-C₄ alkyl.

In another preferred embodiment, the compound of formula (I) above is a compound wherein R¹⁵ is methylene.

In another preferred embodiment, the compound of formula (I) above is a compound wherein R¹⁵ is hydroxy.

In another preferred embodiment, the compound of formula (I) above is a compound wherein R¹⁵ is methoxy or acetoxy.

In another preferred embodiment, the compound of formula (I) above is a compound wherein R¹⁵ is oxo.

In another preferred embodiment, the compound of formula (I) above is a compound wherein R¹⁵ is =NOH.

In another preferred embodiment, the compound of formula (I) above is a compound wherein R¹⁵ is =NOR²³, wherein R²³ is C₁-C₃ alkyl.

In another preferred embodiment, the compound of formula (I) above is a compound wherein R¹⁶ is hydrogen.

In another preferred embodiment, the compound of formula (I) above is a compound wherein R¹⁶ is C₁-C₃ alkyl.

In another preferred embodiment, the compound of formula (I) above is a compound wherein R¹⁶ is methylene.

In another preferred embodiment, the compound of formula (I) above is a compound wherein R¹⁶ is hydroxy.

In another preferred embodiment, the compound of formula (I) above is a compound wherein R¹⁶ is methoxy.

In another preferred embodiment, the compound of formula (I) above is a compound wherein R¹⁶ is oxo.

In another preferred embodiment, the compound of formula (I) above is a compound wherein R¹⁶ is =NOH.

In another preferred embodiment, the compound of formula (I) above is a compound wherein R¹⁶ is =NOR²⁴, wherein R²⁴ is C₁-C₃ alkyl.

In another preferred embodiment, the compound of formula (I) above is a compound wherein R¹⁶, together with R¹⁷, designates an additional bond between the carbon atoms to which R¹⁶ and R¹⁷ are bound.

In another preferred embodiment, the compound of formula (I) above is a compound wherein R¹⁷ is hydrogen.

In another preferred embodiment, the compound of formula (I) above is a compound wherein R¹⁷ is hydroxy.

In another preferred embodiment, the compound of formula (I) above is a compound wherein R¹⁸ and R¹⁹ are both hydrogen.

In another preferred embodiment, the compound of formula (I) above is a compound wherein R¹⁸ and R¹⁹ are both fluoro.

In another preferred embodiment, the compound of formula (I) above is a compound wherein one of R¹⁸ and R¹⁹ is fluoro and the other is hydrogen.

In another preferred embodiment, the compound of formula (I) above is a compound wherein R²⁵ is hydrogen.

In another preferred embodiment, the compound of formula (I) above is a compound wherein R²⁵ is C₁-C₄ alkyl.

In another preferred embodiment, the compound of formula (I) above is a compound wherein R²⁵ is methylene.

In another preferred embodiment, the compound of formula (I) above is a compound wherein R²⁵ is hydroxy.

In another preferred embodiment, the compound of formula (I) above is a compound wherein R²⁵ is oxo.

In another preferred embodiment, the compound of formula (I) above is a compound wherein A is a carbon atom.

In another preferred embodiment, the compound of formula (I) above is a compound wherein A is a carbon atom and R⁷ is hydrogen.

In another preferred embodiment, the compound of formula (I) above is a compound wherein A is a carbon atom R⁷ is hydroxy.

In another preferred embodiment, the compound of formula (I) above is a compound wherein A is a carbon atom R⁷ is fluoro.

In another preferred embodiment, the compound of formula (I) above is a compound wherein A is a carbon atom R⁷, together with R⁸, designates an additional bond between the carbon atoms to which R⁷ and R⁸ are bound.

In another preferred embodiment, the compound of formula (I) above is a compound
wherein A is a carbon atom R⁸ is hydrogen.

In another preferred embodiment, the compound of formula (I) above is a compound wherein A is a carbon atom R⁸ is C₁-C₄ alkyl.

In another preferred embodiment, the compound of formula (I) above is a compound wherein A is a carbon atom R⁸ is methylene.

In another preferred embodiment, the compound of formula (I) above is a compound wherein A is a carbon atom R⁸ is halogen.

In another preferred embodiment, the compound of formula (I) above is a compound wherein A is a carbon atom R²⁰ is C₁-C₄ alkyl.

In another preferred embodiment, the compound of formula (I) above is a compound wherein A is a carbon atom R²⁰ is trifluoromethyl.

In another preferred embodiment, the compound of formula (I) above is a compound wherein A is a carbon atom R²⁰ is C₃-C₆ cycloalkyl.

In another preferred embodiment, the compound of formula (I) above is a compound wherein A is a carbon atom R²¹ is C₁-C₄ alkyl.

In another preferred embodiment, the compound of formula (I) above is a compound wherein A is a carbon atom R²¹ is C₁-C₄ hydroxyalkyl.

In another preferred embodiment, the compound of formula (I) above is a compound wherein A is a carbon atom R²¹ is C₁-C₄ haloalkyl containing up to three halogen atoms.

In another preferred embodiment, the compound of formula (I) above is a compound wherein A is a carbon atom R²¹ is acetoxymethyl.

In another preferred embodiment, the compound of formula (I) above is a compound wherein A is a carbon atom R²¹ is methoxymethyl.

In another preferred embodiment, the compound of formula (I) above is a compound wherein A is a carbon atom and R²¹ is C₃-C₆ cycloalkyl.

In another preferred embodiment, the compound of formula (I) above is a compound wherein A is a carbon atom and R²⁰ and R²¹, together with the carbon atom to which they are bound, form a C₃-C₆ cycloalkyl ring, preferably a cyclopropyl ring, a cyclopentyl ring or a cyclohexyl ring.

In another preferred embodiment, the compound of formula (I) above is a compound wherein A is a nitrogen and R⁷ designates a lone pair of electrons.

In another preferred embodiment, the compound of formula (I) above is a compound wherein A is a nitrogen atom, R⁷ designates a lone pair of electrons and R⁸ is hydrogen.

In another preferred embodiment, the compound of formula (I) above is a compound wherein A is a nitrogen atom, R⁷ designates a lone pair of electrons and R⁸ is C₁-C₄ alkyl.

In another preferred embodiment, the compound of formula (I) above is a compound wherein A is a nitrogen atom, R⁷ designates a lone pair of electrons and R⁸ is oxo.

In another preferred embodiment, the compound of formula (I) above is a compound wherein A is a nitrogen atom, R⁷ designates a lone pair of electrons and R²⁰ and R²¹, independently, are selected from the group comprising C₁-C₄ alkyl, cyclopropyl, cyclopentyl and cyclohexyl.

In a further preferred aspect, the present invention relates to the use of a 15 specific compounds mentioned in claim 1 below as a medicament, in particular as a medicament for use in the regulation of meiosis. The compound may be used neat or in the form of a liquid or solid composition containing auxiliary ingredients conventionally used in the art.

In the present context, the expression "regulating the meiosis" is used to indicate that certain of the compounds of the invention can be used for stimulating the meiosis *in vitro, in vivo,* or *ex vivo*. Thus, the compounds which may be agonists of a naturally occurring meiosis activating substance, can be used in the treatment of infertility which is due to insufficient stimulation of meiosis in females and in males. Other compounds of the invention, which may be antagonists of a naturally occurring meiosis activating substance, can be used for regulating the meiosis, preferably *in vivo*, in a way which makes them suited as contraceptives. In this case the "regulation" means partial or total inhibition.

In a still further preferred aspect, the present invention relates to the use *ex vivo* of a compound of formula (I) above in the regulation of the meiosis of an oocyte, in particular a mammalian oocyte, more particularly a human oocyte.

In a still further preferred aspect, the present invention relates to the use *ex vivo* of a compound of formula (I) above in the stimulation of the meiosis of an oocyte, in particular a mammalian oocyte, more particularly a human oocyte.

In a still further preferred aspect, the present invention relates to the use *ex vivo* of a compound of formula (I) above in the inhibition of the meiosis of an oocyte, in particular a mammalian oocyte, more particularly a human oocyte.

In a still further preferred aspect, the present invention relates to the use *ex vivo* of a compound of formula (I) above in the regulation of the meiosis of a male germ cell, in particular a mammalian male germ cell, more particularly a human male germ cell.

In a still further preferred aspect, the present invention relates to the use *ex vivo* of a compound of formula (I) above in the stimulation of the meiosis of a male germ cell, in particular a mammalian male germ cell, more particularly a human male germ cell.

In a still further preferred aspect, the present invention relates to the use *ex vivo* of a compound of formula (I) above in the inhibition of the meiosis of a male germ cell, in particular a mammalian male germ cell, more particularly a human male germ cell.

In a yet still further preferred aspect, the present invention relates to a method of regulating the meiosis in a mammalian germ cell which method comprises *ex vivo* administering an effective amount of a compound of formula (I) above to a germ cell in need of such a treatment.

In a still further aspect, the present invention relates to a method wherein the germ cell the meiosis of which is to be regulated by means of a compound of formula (I) above is an oocyte.

In a still further aspect, the present invention relates to a method of regulating the meiosis in an oocyte wherein a compound of formula (I) above is administered to the oocyte *ex vivo*.

In a still further aspect, the present invention relates to a method of regulating the meiosis of a male germ cell by administering *ex vivo* a compound of formula (I) above to the cell.

In a still further aspect, the present invention relates to a method whereby mature male germ cells are produced by administering *in vitro* a compound of formula (I) above to testicular tissue containing immature cells.

### DETAILED DESCRIPTION OF THE INVENTION

As used in the present description and claims, the expression C₁-C₃ alkyl designates an alkyl group having from one to three carbon atoms; preferred examples are methyl, ethyl and propyl, more preferred methyl and ethyl. Similarly, the expression C₁-C₄ alkyl designates an alkyl group having from one to four carbon atoms; preferred examples are methyl, ethyl, propyl, isopropyl and butyl, more preferred methyl and ethyl. The expression C ₁-C₆ alkyl designates an alkyl group having from one to six carbon atoms; preferred examples are methyl, ethyl, propyl, isopropyl, butyl, *tert*-butyl, pentyl and hexyl, more preferred methyl, ethyl, propyl, isopropyl, butyl and *tert*-butyl, still more preferred methyl and ethyl.

As used in the present description and claims, the expression C₁-C₃ alkoxy designates an alkoxy group having from one to three carbon atoms; preferred examples are methoxy, ethoxy and propoxy, more preferred methoxy and ethoxy.

As used in the present description and claims, the expression halogen preferably designates fluoro and chloro, more preferred fluoro.

The compounds dealt with herein have a number of chiral centres in the molecule and thus exists in several isomeric forms. All these isomeric forms and mixtures thereof are within the scope of the invention.

The compounds dealt with herein will influence the meiosis in oocytes as well as in male germ cells.

The existence of a meiosis inducing substance in nature has been known for some time. However, until recently the identity of the meiosis inducing substance or substances was unknown.

The prospects of being able to influence the meiosis are several. According to a preferred embodiment of the present invention, the compounds dealt with herein are used to stimulate the meiosis. According to another preferred embodiment of the present invention, the compounds dealt with herein are used to stimulate the meiosis in humans. Thus, the compounds dealt with herein are promising as new fertility regulating agents without the usual side effect on the somatic cells which are known from the hitherto used hormonal contraceptives which are based on estrogens and/or gestagens.

For use as a contraceptive agent in females, a meiosis inducing substance can be administered so as to prematurely induce resumption of meiosis in oocytes while they are still in the growing follicle, before the ovulatory peak of gonadotropins occurs. In women, the resumption of the meiosis can, for example, be induced a week after the preceding menstruation has ceased. When ovulated, the resulting overmature oocytes are then most likely not to be fertilized. The normal menstrual cycle is not likely to be affected. In this connection it is important to notice, that the biosynthesis of progesterone in cultured human granulosa cells (somatic cells of the follicle) is not affected by the presence of a meiosis inducing substance whereas the estrogens and gestagens used in the hitherto used hormonal contraceptives do have an adverse effect on the biosynthesis of progesterone.

According to another aspect of this invention, the meiosis indudng substance dealt with herein can be used in the treatment of certain cases of infertility in females, including women, for example, by administration thereof to the oocytes of a females who, due to an insufficient own production of meiosis activating substance, are unable to produce mature oocytes. Hence, when *in vitro* fertilization is performed, better results are achieved, when a compound dealt with herein is added to the medium in which the oocytes are kept.

When infertility in males, including men, is caused by an insufficient own production of the meiosis activating substance and thus a lack of mature sperm cells, use of a compound dealt with herein may relieve the problem..

As an alternative to the method described above, contraception in females can also be achieved by use of a compound dealt with herein which inhibits the meiosis, so that no mature oocytes are produced. Similarly, contraception in males can be achieved by use of a compound dealt with herein which inhibits the meiosis, so that no mature sperm cells are produced.

The route of administration of compositions containing a compound of claim 1 may be any route which effectively transports the active compound to its site of action.

Thus, when the compounds dealt with herein are to be administered to a mammal, they are conveniently provided in the form of a pharmaceutical composition which comprises at least one compound dealt with herein in connection with a pharmaceutically acceptable carrier. For oral use, such compositions are preferably in the form of capsules or tablets.

From the above it will be understood that administrative regimen called for will depend on the condition to be treated. Thus, when used in the treatment of infertility the administration may have to take place once only, or for a limited period, e.g. until pregnancy is achieved. When used as a contraceptive, the compounds dealt with herein will either have to be administered continuously or cyclically. When used as a contraceptive by females and not taken continuously, the timing of the administration relative to the ovulation will be important.

Pharmaceutical compositions comprising a compound dealt with herein may further comprise carriers, diluents, absorption enhancers, preservatives, buffers, agents for adjusting the osmotic pressure, tablet disintegrating agents and other ingredients which are conventionally used in the art. Examples of solid carriers are magnesium carbonate, magnesium stearate, dextrin, lactose, sugar, talc, gelatin, pectin, tragacanth, methyl cellulose, sodium carboxymethyl cellulose, low melting waxes and cocoa butter.

Liquid compositions include sterile solutions, suspensions and emulsions. Such liquid compositions may be suitable for injection or for use in connection with *ex vivo* and *in vitro* fertilization. The liquid compositions may contain other ingredients which are conventionally used in the art, some of which are mentioned in the list above.

Further, a composition for transdermal administration of a compound of this invention may be provided in the form of a patch and a composition for nasal administration may be provided in the form of a nasal spray in liquid or powder form.

The dose of a compound of the invention to be used will be determined by a physician and will depend, *inter alia*, on the particular compound employed, on the route of administration and on the purpose of the use.

The compounds dealt with herein can be synthesized by methods known per se.

The present invention is further illustrated by the following examples which, however, are not to be construed as limiting the scope of protection. The features disclosed in the foregoing description and in the following examples may, in any combination thereof, be material for realising the invention in diverse forms thereof.

### EXAMPLES

### EXAMPLE 1

### Preparation of 7-oxo-5α-cholest-8-ene-3β-ol.

0.50 g of 3β-acetoxy-7-oxo-5α-cholest-8-ene (Fieser, LF *J Am Chem Soc* (1953) 4395) was refluxed in a mixture of 30 ml of ethanol and 20 ml of 1M aqueous sodium hydroxide for 1 hour. After cooling to room temperature, 23 ml of 1 M hydrochloric acid and 100 ml of water were added. After cooling on an ice bath, the precipitate was filtered off, washed with water and dried to give 0.435 g of the crude compound which was purified by chromatography on silica gel (methylene chloride/methanol, 40:1 (w/w)) and crystallized from methanol/water to give 0.198 g of the title compound.
Melting point: 115-117° C.
The ¹H-NMR spectrum (CDCl₃,d) showed characteristic signals at: 0.59 (s,3H); 1.18 (s,3H); 3.64 (m,1H).
The ¹³C-NMR spectrum (CDCl₃, 100.6 MHz) showed characteristic signals at: 69.5; 132.8; 164.8; 198.6.

### EXAMPLE 2

### Preparation of 7-oxo-5α-cholesta-8,14-diene-3β-ol.

The compound was prepared as described by Fieser, LF *et al. J Am Chem Soc* (1953) 4719) and showed the following characteristic physical constants:
Melting point: 140-142° C.
¹H-NMR spectrum (CDCl₃,d): 0.79 (s,3H), 1.14 (s,3H), 3.66 (m,1H), 6.45 (s,1H).
¹³C-NMR spectrum (CDCl₃, 100.6 MHz): 69.4; 126.1; 126.6; 140.8; 164.9; 197.2.

### EXAMPLE 3

### Preparation of 7α-methyl-5a-cholest-8-ene-3b,7b-diol.

0.50 g of 3b-acetoxy-7-oxo-5α-cholest-8-ene (Fieser, LF *J Am Chem Soc* (1953) 4395) was dissolved in 10 ml of tetrahydrofuran and 3 ml of 3M methylmagnesium chloride in tetrahydrofuran was added dropwise at 0° C over 15 minutes. The mixture was stirred at room temperature for 1 hour, cooled to 0° C, and 50 ml of a 1M solution of ammonium chloride was added dropwise over 5 minutes. The mixture was extracted twice with 50 ml of ethylacetate. The combined organic phases were washed with water and brine and evaporated to yield 474 mg of the crude product which was crystallized from ethylacetate/heptane to yield 168 mg of the title compound.
Melting point: 92-94° C.
The ¹H-NMR spectrum (CDCl₃,d) showed characteristic signals at: 0.69 (s,3H), 1.03 (s,3H), 1.37 (s,3H), 3.62 (m,1H).
The ¹³C-NMR spectrum (CDCl₃, 50.3 MHz) showed characteristic signals at: 70.7; 73.8; 132.9; 139.2.

From the mother liquor another crop (107 mg) of the title compound was isolated.

### EXAMPLE 4

### Preparation of 11-oxo-5α-cholest-8-ene-3β-ol.

This compound was prepared as described by Parish, ES *et al. Steroids* **48** (1986) 407) and showed physical constants as described in the literature.

### EXAMPLE 5

### Preparation of 3β-Hydroxy-5α-cholest-8-ene-7-oxime.

0.25 g of 7-oxo-5α-cholest-8-ene-3β-ol (cf. Example 1) was dissolved in 10 ml of dry pyridine. 0.43 g of hydroxylamine hydrochloride was added, and the mixture was stirred at 70° C for 3 hours. After evaporation to dryness, the residue was triturated with water to give 238 mg of the crude product. Recrystallization from methanol yielded 164 mg of the title compound.
Melting point: 218-223° C.
The ¹H-NMR spectrum (CDCl₃,d) showed characteristic signals at: 0.62 (s,3H), 1.03 (s,3H), 3.0 (dd,1H), 3.62 (m,1H), 7.52 (broad s,1H).
The ¹³GNMR spectrum (CDCl₃, 100.6 MHz) showed characteristic signals at: 69.9, 126.7, 149.8, 157.7.

### EXAMPLE 6

### Preparation of 3β-acetoxy-7-oxo-5α-cholest-8-ene.

This compound was prepared as described by Fieser, LF *J Am Chem Soc* (1953) 4395 and showed physical constants as described in the literature.

### EXAMPLE 7

### Preparation of 3β-acetoxy-7-oxo-5α-cholesta-8,14-diene.

This compound was prepared as described by Fieser, LF *et al. J Am Chem Soc* (1953) 4719 and showed physical constants as described in the literature.

### EXAMPLE 8

### Preparation of 7-oxo-5α-cholest-8-ene-3β-yl benzoate.

This compound was prepared as described by Parish EJ *et al. Steroids* **48** (1986) 407 and showed physical constants as described in the literature.

### EXAMPLE 9

### Preparation of 7-methylene-5α-cholest-9-ene-3β-ol.

0.54 g of sodium hydride (60%) was dissolved in 10 ml of dimethyl sulfoxide at 70° C. After 15 minutes a solution of 5.24 g of methyltriphenylphosphonium bromide in 33 ml of dimethyl sulfoxide and then a solution of 3β-acetoxy-7-oxo-5α-cholest-8-ene (cf. Example 6) in 28 ml benzene was added. The mixture was stirred at 60° C for 22 hours, cooled to room temperature, poured on 1M hydrochloride acid/ice, and extracted several times with benzene. The combined organic phases were evaporated to dryness and the residue was dissolved in a mixture of methanol/water/cyclohexane, 13:7:20 (w/w). The methanol/water phase was extracted several times with cyclohexane and the combined cyclohexane phases were evaporated to dryness to give 1.32 g of an oil which was dissolved in 15 ml of heptane, filtered and evaporated to dryness. The residue (0.80 g) was chromatographed on 40 g silica gel (toluene/ethylacetate, 9:1 (w/w)) to give 247 mg of an almost pure product, which was crystallized from methanol to yield 110 mg of the title compound.
Melting point: 44-50° C.
The ¹H-NMR spectrum (CDCl₃,d) showed characteristic signals at: 0.65 (s,3H); 1.06 (s,3H); 2.62 (d,1H); 3.58 (m,1H); 4.68 (d,2H); 5.27 (d,1H).
The ¹³C-NMR spectrum (CDCl₃, 100.6 MHz) showed characteristic signals at: 70.5; 105.2; 115.7; 146.1; 150.5.

### EXAMPLE 10

### Preparation of 7-methyl-5α-cholesta-6,8-diene-3β-ol.

0.90 g of 7α-methyl-5α-cholest-8-ene-3β,7β-diol (cf. Example 3) was suspended in 55 ml of formic acid and stirred overnight at room temperature. The mixture was poured on ice water and the precipitated compound was filtered off, washed with water, and dried. The residue (0.84 g) was refluxed in a mixture of 50 ml ethanol and 25 ml 1M aqueous sodium carbonate for 15 minutes. The solvent was evaporated and the residue was redissolved in methylene chloride and water. The organic phase was evaporated to dryness and crystallized from ethanol/water to yield 395 mg of the title compound.
Melting point: 112-113° C.
The ¹H-NMR spectrum (CDCl₃,d) of the product showed characteristic signals at: 0.58 (s,3H), 0.88 (s,3H), 1.83 (s.3H), 3.58 (m,1H), 5.37 (d,1H).
The ¹³C-NMR spectrum (CDCl₃, 100.6 MHz) showed characteristic signals at: 70.9, 116.6, 129.0, 129.6, 145.3.

### EXAMPLE 11

### Preparation of 11-oxo-5α-cholest-8-ene-3β-yl benzoate.

This compound was prepared as described by Parish, EJ *et al. Steroids* **48** (1986) 407) and showed physical constants as described in literature.

### EXAMPLE 12

### Preparation of cholesta-8,14-diene-5α-H-3-one.

Cholesta-8,14-diene-5α-3-one was prepared according to Dolle *J Org Chem* **51** (1986) 4047-4053. The product showed the following physical characteristics:
¹H-NMR: Hd: 5.78 (d 1H, C4H), 5.16 (1H, m, C7H)
Elementary analysis:
Cal: C: 84.7; H: 11.1; O: 4.18
Found: C: 84.7; H: 11.4.

### EXAMPLE 13

### Preparation of 3α-flourocholesta-8,14-diene.

Cholesta-8,14-diene-3β-ol (1.17 g, 3 mmol) was dissolved in 10 ml of methylenechloride and cooled to -78° C. Over 10 min a solution of diethylaminosulfur trifluoride (1.4 g, 8.7 mmol) in 10 ml of methylenechloride was added at -78° C. The mixture was stirred for 1 1/2 hour at -78° C and was then slowly heated to room temperature. To the reaction mixture was added 15 ml of water while stirring was continued. The organic phase was separated and washed with 30 ml of 5% NaHCO₃ and then with water. The organic phase was dried with MgSO₄ and evaporated to dryness. The residue was purified by column chromatography using heptane for a first fraction and heptane/acetone, 95:5 (w/w) for a second fraction containing 3α-fluorocholesta-8.14 diene, 0.14 g (12%).
Melting point: 98.6° C
Elementary analysis:
Cal C: 83.88; H: 11.21; F: 4.91.
Found C: 83.92; H: 11.75.
¹⁹F-NMR: d 181.0 and 181.2 (J_{HCF} 45.2 Hz, C₃-aF).

### EXAMPLE 14

### Preparation of cholesta-2,8,14-triene.

The title compound was prepared by using a method analogous to a method described in *J Chemical Research* (miniprint) (1979) 4714-4755.
Cholesta-8,14-diene-3β-ol (1.17 g, 3 mmol) was dissolved in 10 ml of methylenechloride and cooled to -78° C. Over 10 min a solution of diethylaminosulfur trifluoride (1.4 g, 8,7 mmol) in 10 ml of methylenechloride was added at -78° C. The mixture was stirred and was then slowly heated to the room temperature. The reaction mixture was added 15 ml water while stirring was continued. The organic phase was separated and washed with 30 ml of 5% NaHCO₃ and then with water. The organic phase was dried with MgSO₄ and evaporated to dryness. The residue was purified by column chromatography using heptane for a first fraction A giving cholesta-2,8,14-triene, 0.23 g.
Melting point: 104.7° C.
Elementary analysis:
Cal C: 88.45; H: 11.55.
Found C: 88.58; H: 11.89.
NMR: Hd: 5.64 (m 2H; C₂-H; C₃-H)d 5.35 (s, 1H C 15H).
Cd: 125.95 (C₃), 125.67 (C₂).

### EXAMPLE 15

### Preparation of cholesta-8,14-diene-5α(H)-3-(E),(Z)-oxime.

Cholesta-8,14-diene-3-one (1.0 g, 2.61 mmol) was dissolved in 15 ml of pyridine and hydroxylamine, HCl (0.29 g, 4.23 mmol) was added. The reaction mixture was heated at 70-72° C for 1 1/2 hour while stirred. The reaction mixture was cooled and evaporated to dryness. 30 ml of 50% acetic acid/water was added and the crystals formed were separated by filtration. The crystals were dissolved in heptane and washed with water. The organic phase was separated and evaporated to dryness. The crystals were recrystallized from ethanol to give 0.91 g of 5α-cholesta-8,14-diene-3-(E) and (Z)-oxime.
Elementary analysis:
Cal C: 81.55; H: 10.90; N: 3.52; O: 4.02.
Found: 81.65; H: 11.30; N: 3.43.
¹³C-NMR: d 159,66 and 159.51 (3-C).

## Claims

1. The use of one of the following compounds 7-oxo-5α-cholesta-8,14-diene-3β-ol; 7α-methyl-5α-cholest-8-ene-3β,7β-diol; 3β-Hydroxy-5α-cholest-8-ene-7-oxime; 3β-acetoxy-7-oxo-5α-cholest-8-ene; 3β-acetoxy-7-oxo-5α-cholesta-8,14-diene; 7-methytene-5α-cholest-9-ene-3β-ol; 7-methyl-5α-cholesta-6,8-diene-3β-ol; cholesta-8,14-diene-5α-H-3-one; 3α-flourocholesta-8,14-diene; cholesta-2,8,14-triene; and cholesta-8,14-diene-5α(H)-3-(E),(Z)-oxime as a medicament.

2. The use of a compound of the general formula (I) 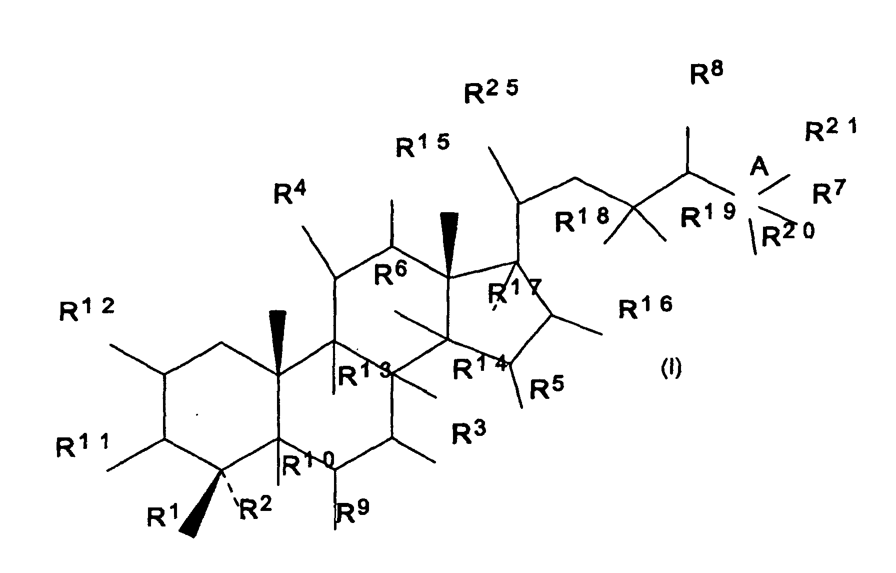 wherein R¹ and R², independently, are selected from the group comprising hydrogen and branched or unbranched C₁-C₆ alkyl which may be substituted by halogen, hydroxy or cyano, or wherein R¹ and R² together designate methylene or, together with the carbon atom to which they are bound, form a cyclopropane ring, a cyclopentane ring, or a cyclohexane ring; R³ is selected from the group comprising hydrogen, methylene, hydroxy, methoxy, acetoxy, oxo, =NOR²⁶ wherein R²⁶ is hydrogen or C₁-C₃ alkyl, halogen, and hydroxy and C₁-C₄ alkyl bound to the same carbon atom of the sterol skeleton, or R³ designates, together with R⁹ or R¹⁴, an additional bond between the carbon atoms to which R³ and R⁹ or R¹⁴ are bound; R⁴ is selected from the group comprising hydrogen, methylene, hydroxy, methoxy, acetoxy, oxo, =NOR²⁷ wherein R ²⁷ is hydrogen or C₁-C₃ alkyl, halogen, and hydroxy and C₁-C₄ alkyl bound to the same carbon atom of the sterol skeleton, or R⁴ designates, together with R¹³ or R¹⁵, an additional bond between the carbon atoms to which R⁴ and R¹³ or R¹⁵ are bound; R⁵ is selected from the group comprising hydrogen, C₁-C₄ alkyl, methylene, hydroxy, methoxy, oxo, and =NOR²² wherein R²² is hydrogen or C₁-C₃ alkyl, or R⁵ designates, together with R⁶, an additional bond between the carbon atoms to which R⁵ and R⁶ are bound; R⁶ is hydrogen or R⁶ designates, together with R⁵, an additional bond between the carbon atoms to which R⁵ and R⁶ are bound; R⁹ is hydrogen or R⁹ designates, together with R³ or R¹⁰, an additional bond between the carbon atoms to which R⁹ and R³ or R¹⁰ are bound; R¹⁰ is hydrogen or R¹⁰ designates, together with R⁹, an additional bond between the carbon atoms to which R¹⁰ and R⁹ are bound; R¹¹ is selected from the group comprising hydroxy, alkoxy, substituted alkoxy, acyloxy, sulphonyloxy, phosphonyloxy, oxo, =NOR²⁸ wherein R²⁸ is hydrogen or C₁-C₃ alkyl, halogen and hydroxy and C₁-C₄ alkyl bound to the same carbon atom of the sterol skeleton, or R¹¹ designates, together with R¹², an additional bond between the carbon atoms to which R¹¹ and R¹² are bound; R¹² is selected from the group comprising hydrogen, C₁-C₃ alkyl, vinyl, C₁-C₃ alkoxy and halogen, or R¹² designates, together with R¹¹, an additional bond between the carbon atoms to which R¹² and R¹¹ are bound; R¹³ is hydrogen or R¹³ designates, together with R⁴ or R¹⁴, an additional bond between the carbon atoms to which R¹³ and R⁴ or R¹⁴ are bound; R¹⁴ is hydrogen or R¹⁴ designates, together with R³, R⁶ or R¹³, an additional bond between the carbon atoms to which R¹⁴ and R³ or R⁶ or R¹³ are bound; R¹⁵ is selected from the group comprising hydrogen, C₁-C₄ alkyl, methylene, hydroxy, methoxy, acetoxy, oxo, and =NOR²³ wherein R²³ is hydrogen or C₁-C₃ alkyl, or R¹⁵ designates, together with R⁴, an additional bond between the carbon atoms to which R¹⁵ and R⁴ are bound; R¹⁶ is selected from the group comprising hydrogen, C₁-C₃ alkyl, methylene, hydroxy, methoxy, oxo and =NOR²⁴ wherein R²⁴ is hydrogen or C₁-C₃ alkyl, or R¹⁶ designates, together with R¹⁷, an additional bond between the carbon atoms to which R¹⁶ and R¹⁷ are bound; R¹⁷ is hydrogen or hydroxy or R¹⁷ designates, together with R¹⁶, an additional bond between the carbon atoms to which R¹⁷ and R¹⁶ are bound; R¹⁸ and R¹⁹ are, independently, hydrogen or fluoro; R²⁵ is selected from the group comprising hydrogen, C₁₋₄ alkyl, methylene, hydroxy and oxo; A is a carbon atom or a nitrogen atom; when A is a carbon atom, R⁷ is selected from the group comprising hydrogen, hydroxy and fluoro, and R⁸ is selected from the group comprising hydrogen, C₁-C₄ alkyl, methylene and halogen, or R⁷ designates, together with R⁸, an additional bond between the carbon atoms to which R⁷ and R⁸ are bound; R²⁰ is selected from the group comprising C₁-C₄ alkyl, trifluoromethyl and C₃-C₆ cycloalkyl and R²¹ is selected from the group comprising C₁-C₄ alkyl, C₁-C₄ hydroxyalkyl, C₁-C₄ haloalkyl containing up to three halogen atoms, methoxymethyl, acetoxymethyl, and C₃-C₆ cycloalkyl, or R ²⁰ and R²¹, together with the carbon atom to which they are bound, form a C₃-C₆ cycloalkyl ring; and when A is a nitrogen atom, R⁷ designates a lone pair of electrons and R⁸ is selected from the group comprising hydrogen, C₁-C₄ alkyl and oxo; R²⁰ and R²¹ are, independently, C₁-C₄ alkyl or C₃-C₆ cycloalkyl; with the proviso that the compound of the general formula (I) does not have any cumulated double bonds and with the further proviso that the compound is not a compound of the general formula (II) 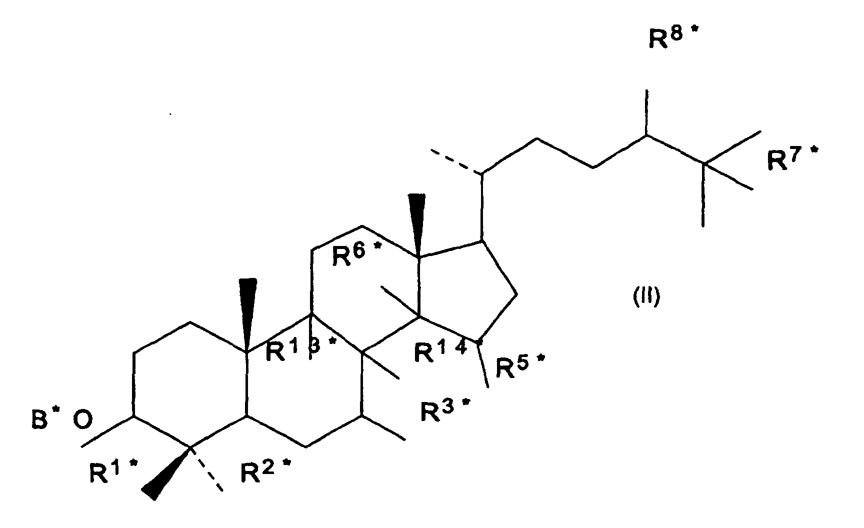 wherein R^{1*} and R^{2*}, independently, are selected from the group comprising hydrogen, branched or unbranched C₁-C₆ alkyl which may be substituted by halogen or hydroxy or wherein R^{1*} and R^{2*}, together with the carbon atom to which they are bound, form a cyclopentane ring or a cyclohexane ring; R^{13*} and R^{14*} together designate an additional bond between the carbon atoms to which they are bound in which case R^{3*} is hydrogen and R^{6*} and R^{5*} are either hydrogen or together they designate an additional bond between the carbon atoms to which they are bound; or R^{3*} and R^{14*} together designate an additional bond between the carbon atoms to which they are bound in which case R^{13*} is hydrogen and R^{6*} and R^{5*} are either hydrogen or together they designate an additional bond between the carbon atoms to which they are bound; or R ^{6*} and R^{14*} together designate an additional bond between the carbon atoms to which they are bound in which case R^{13*}, R^{3*} and R^{5*} are all hydrogen; R^{8*} and R^{7*} are hydrogen or together they designate an additional bond between the carbon atoms to which they are bound; and B* is either hydrogen or an acyl group, including a sulphonyl group or a phosphonyl group, or a group which together with the remaining part of the molecule forms an ether, and esters and ethers thereof for preparing a medicament to be used in the regulation of meiosis.

3. The use according to the previous claim wherein the compound is any of the following compounds: 7-oxo-5α-cholest-8-ene-3β-ol; 7-oxo-5α-cholesta-8,14-diene-3β-ol; 7α-methyl-5α-cholest-8-ene-3β,7β-diol; 11-oxo-5α-chotest-8-ene-3β-ol; 3β-Hydroxy-5α-cholest-8-ene-7-oxime; 3β-acetoxy-7-oxo-5α-cholest-8-ene; 3β-acetoxy-7-oxo-5α-cholesta-8,14-diene; 7-oxo-5α-cholest-8-ene-3β-yl benzoate; 7-methylene-5α-cholest-9-ene-3β-ol; 7-methyl-5α-cholesta-6,8-diene-3β-ol; 11-oxo-5α-cholest-8-ene-3β-yl benzoate; cholesta-8,14-diene-5α-H-3-one; 3α-flourocholesta-8,14-diene; cholesta-2,8,14-triene; and cholesta-8,14-diene-5α(H)-3-(E),(Z)-oxime.

4. A method of regulating the meiosis in a mammalian germ cell which method comprises administering *ex vivo* an effective amount of a compound of formula (I) stated in Claim 2, preferably a compound stated in claim 3, to a germ cell in need of such a treatment.

5. A method according to the previous claim wherein the germ cell the meiosis of which is to be regulated is an oocyte.

6. A method according to claim 4 wherein a compound of formula (I) stated in Claim 2, preferably a compound stated in claim 3, is administered to an oocyte *ex vivo*.

7. A method according to claim 4 wherein the germ cell the meiosis of which is to be regulated is a male germ cell.

8. A method according to claim 4 whereby mature male germ cells are produced by administering a compound of formula (I) stated in Claim 2, preferably a compound stated in claim 3, to testicular tissue *in vitro.*

## Patentansprüche

1. Die Verwendung von einer der folgenden Verbindungen 7-Oxo-5α-cholesta-8,14-dien-3β-ol; 7α-Methyl-5α-cholest-8-en-3β, 7β-Diol; 3β-Hydroxy-5α-cholest-8-en-7-oxim; 3β-Acetoxy-7-oxo-5α-cholest-8-en; 3β-Acetoxy-7-oxo-5α-cholesta-8,14-dien; 7-Methylen-5α-cholest-9-en-3β-ol; 7-Methyl-5α-cholesta-6,8-dien-3β-ol; Cholesta-8,14-dien-5α-H-3-on; 3α-Fluorcholesta-8,14-dien; cholesta-2,8,14-trien; und cholesta-8,14-dien-5α(H)-3(E), (Z)-Oxim als Arzneimittel.

2. Die Verwendung einer Verbindung der allgemeinen Formel (I) 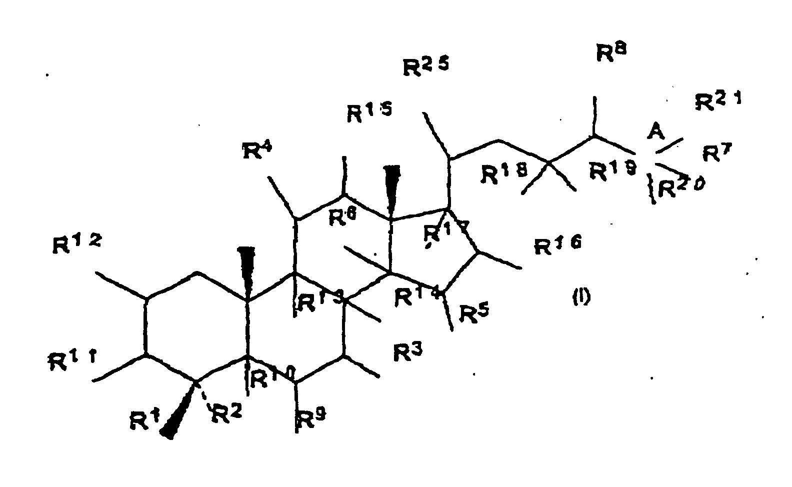 wobei R¹ und R², unabhängig von einander, ausgewählt werden, aus der Gruppe, umfassend Wasserstoff und verzweigtes oder unverzweigtes C₁-C₆-Alkyl, welche mit Halogen, Hydroxy oder Cyan substituiert werden können, oder wobei R¹ und R² gemeinsam Methylen darstellen, oder zusammen mit dem Kohlenstoffatom, an welches sie gebunden sind, einen Cyclopropanring, einen Cyclopentanring oder einen Cyclohexanring bilden; R³ wird ausgewählt aus der Gruppe, umfassend Wasserstoff, Methylen, Hydroxy, Methoxy, Acetoxy, Oxo, =NOR²⁶, wobei R²⁶ ein Wasserstoff ist oder ein C₁-C₃-Alkyl, Halogen, und Hydroxy, und C₁-C₄-Alkyl, an das gleiche Kohlenstoffatom des Sterolskeletts gebunden ist, oder R³ bezeichnet, zusammen mit R⁹ oder R¹⁴, eine zusätzliche Bindung zwischen den Kohlenstoffatomen, an welche R³ und R⁹ oder R¹⁴ gebunden sind; R⁴ ist ausgewählt aus der Gruppe, umfassend Wasserstoff, Methylen, Hydroxy, Methoxy, Acetoxy, Oxo, =NOR²⁷, wobei R²⁷ Wasserstoff ist oder C₁-C₃-Alkyl, Halogen, und Hydroxy und C₁-C₄-Alkyl, an das gleiche Kohlenstoffatom des Sterolskeletts gebunden, oder R⁴ bezeichnet, zusammen mit R¹³ oder R¹⁵, eine zusätzliche Bindung zwischen den Kohlenstoffatomen, an welche R⁴ und R¹³ oder R¹⁵ gebunden sind; R⁵ ist aus einer Gruppe ausgewählt, umfassend Wasserstoff, C₁-C₄ Alkyl, Methylen, Hydroxy, Methoxy, Oxo, und =NOR²², worin R²² Wasserstoff oder C₁-C₃ Alkyl ist, oder R⁵ bezeichnet, zusammen mit R⁶, eine zusätzliche Bindung zwischen den Kohlenstoffatomen, an welche R⁵ und R⁶ gebunden sind; R⁶ ist Wasserstoff oder R⁶ bezeichnet, zusammen mit R⁵ eine zusätzliche Bindung zwischen den Kohlenstoffatomen, an welche R⁵ und R⁶ gebunden sind; R⁹ ist Wasserstoff oder R⁹ bezeichnet, zusammen mit R³ oder R¹⁰, eine zusätzliche Bindung zwischen den Kohlenstoffatomen, an welche R⁹ und R³ oder R¹⁰ gebunden sind; R¹⁰ ist Wasserstoff oder R¹⁰ bezeichnet, zusammen mit R⁹, eine zusätzliche Bindung zwischen den Kohlenstoffatomen, an welche R¹⁰ und R⁹ gebunden sind; R¹¹ ist ausgewählt aus der Gruppe, umfassend Hydroxy, Alkoxy, substituierte Alkoxy, Acyloxy, Sulphonyloxy, Phosphonyloxy, Oxo, =NOR²⁸, wobei R²⁸ Wasserstoff ist oder C₁-C₃ Alkyl, Halogen und Hydroxy und C₁-C₄ Alkyl an das gleiche Kohlenstoffatom des Scerolskeletts gebunden, oder R¹¹ bezeichnet, zusammen mit R¹², eine zusätzliche Bindung zwischen den Kohlenstoffatomen, an welche R¹¹ und R¹² gebunden sind; R¹² ist ausgewählt aus der Gruppe, umfassend Wasserstoff, C₁-C₃ Alkyl, Vinyl, C₁-C₃ Alkoxy und Halogen, oder R¹² bezeichnet, zusammen mit R¹¹, eine zusätzliche Bindung zwischen den Kohlenstoffatomen, an welche R¹² und R¹¹ gebunden sind; R¹³ ist Wasserstoff oder R¹³ bezeichnet, zusammen mit R⁴ oder R¹⁴, eine zusätzliche Bindung zwischen den Kohlenstoffatomen, an welche R¹³ und R⁴ oder R¹⁴ gebunden sind; R¹⁴ ist eine Wasserstoff oder R¹⁴ bestimmt zusammen mit R³, R⁶ oder R¹³, eine zusätzliche Bindung zwischen den Kohlenstoffatomen, an welche R¹⁴ und R³ oder R⁶ oder R¹³ gebunden sind; R¹⁵ ist ausgewählt aus der Gruppe, umfassend Wasserstoff, C₁-C₄ Alkyl, Methylen, Hydroxy, Methoxy, Oxo und =NOR²³, wobei R²³ ein Wasserstoff oder C₁-C₃ Alkyl ist, oder R¹⁵ bezeichnet, zusammen mit R⁴, eine zusätzliche Bindung zwischen den Kohlenstoffatomen, an welche R¹⁵ und R⁴ gebunden sind; R¹⁶ wird ausgewählt aus der Gruppe, umfassend Wasserstoff, C₁-C₃ Alkyl, Methylen, Hydroxy, Methoxy, Oxo und =NOR²⁴, wobei R²⁴ ein Wasserstoff oder C₁-C₃ Alkyl ist, oder R¹⁶ bezeichnet, zusammen mit R¹⁷, eine zusätzliche Bindung zwischen den Kohlenstoffatomen, an welche R¹⁶ und R¹⁷ gebunden sind; R¹⁷ ist Wasserstoff oder Hydroxy oder R¹⁷ bezeichnet, zusammen mit R¹⁶, eine zusätzliche Bindung zwischen den Kohlenstoffatomen, an welche R¹⁷ und R¹⁶ gebunden sind; R¹⁸ und R¹⁹ sind, unabhängig voneinander, Wasserstoff oder Fluor, R²⁵ ist ausgewählt aus der Gruppe, umfassend Wasserstoff, C₁₋₄ Alkyl, Methylen, Hydroxy und Oxo; A ist ein Kohlenstoffatom oder ein Stickstoffatom; falls A ein Kohlenstoffatom ist, wird R⁷ ausgewählt aus der Gruppe, umfassend Wasserstoff, Hydroxy und Flour, und R⁸ wird ausgewählt aus der Gruppe, umfassend Wasserstoff, C₁-C₄ Alkyl, Methylen und Halogen, oder R⁷ bezeichnet, zusammen mit R⁸, eine zusätzliche Bindung zwischen den Kohlenstoffatomen, an welche R⁷ und R⁸ gebunden sind; R²⁰ wird ausgewählt aus der Gruppe, umfassend C₁-C₄ Alkyl, Trifluormethyl und C₃-C₆ Cycloalkyl und R²¹ wird ausgewählt aus der Gruppe, umfassend C₁-C₄ Alkyl, C₁-C₄ Hydroxyalkyl, C₁-C₄ Haloalkyl, enthalten bis zu drei Halogenatome, Methoxymethyl, Acetoxymethyl, und C₃-C₆ Cycloalkyl, oder R²⁰ und R²¹ bilden zusammen mit dem Kohlenstoffatom, an welches sie gebunden sind, einen C₃-C₆ Cycloalkylring; und falls A ein Stickstoffatom ist, bezeichnet R⁷ ein einziges Elektronenpaar und R⁸ wird ausgewählt aus der Gruppe, umfassend Wasserstoff, C₁-C₄ Alkyl und Oxo; R²⁰ und R²¹ sind, unabhängig von einander, C₁-C₄ Alkyl oder C₃-C₆ Cycloalkyl; mit der Bedingung, dass die Verbindung der allgemeinen Formel (I) nicht kumulierte Doppelbindungen hat und mit der weiteren Bedingung, dass die Verbindung nicht eine Verbindung der allgemeinen Formel (II) 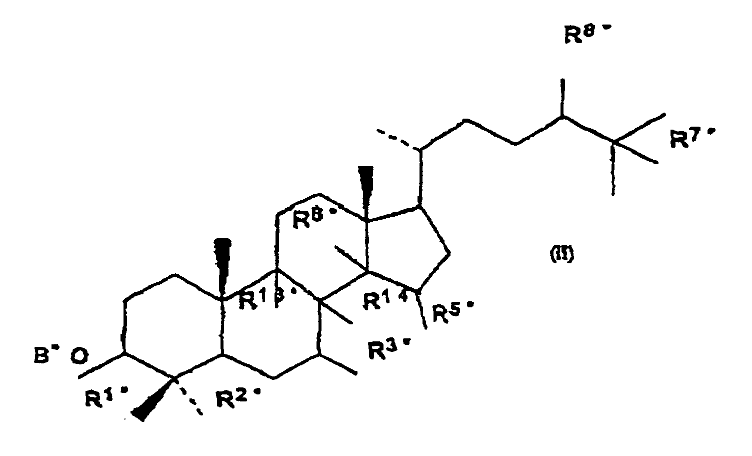 ist, wobei R¹* und R²*, unabhängig von einander, ausgewählt sind aus der Gruppe, umfassend Wasserstoff, verzweigtes oder unverzweigtes C₁-C₆ Alkyl, welches mit Halogen oder Hydroxy substituiert werden kann, oder wobei R¹* und R²*, zusammen mit dem Kohlenstoffatom, an welches sie gebunden sind, einen Cyclopentanring oder Cyclohexanring bilden; R¹³* und R¹⁴* bezeichnen gemeinsam eine zusätzliche Bindung zwischen den Kohlenstoffatomen, an welche sie gebunden sind, in welchem Fall R³* ein Wasserstoff ist und R⁶* und R⁵* entweder Wasserstoff sind oder gemeinsam eine zusätzliche Bindung zwischen den Kohlenstoffatomen, an welche sie gebunden sind, bezeichnen; oder R³* und R¹⁴* bezeichnen gemeinsam eine zusätzliche Bindung zwischen den Kohlenstoffatomen, an welche sie gebunden sind, in welchem Falle R¹³* Wasserstoff ist und R⁶* und R⁵* entweder Wasserstoff sind, oder sie bezeichnen gemeinsam eine zusätzliche Bindung zwischen den Kohlenstoffatomen, an welche sie gebunden sind; oder R⁶* und R¹⁴* bezeichnen gemeinsam eine zusätzliche Bindung zwischen den Kohlenstoffatomen, an welche sie gebunden sind, in welchem Falle R¹³*, R³* und R⁵* alle Wasserstoff sind; R⁸* und R⁷* sind Wasserstoff oder bezeichnen gemeinsam eine zusätzliche Bindung zwischen den Kohlenstoffatomen, an welche sie gebunden sind; und B* ist entweder Wasserstoff oder eine Acylgruppe, einschließend eine Sulphonylgruppe oder eine Phosphonylgruppe, oder eine Gruppe, welche zusammen mit dem übrig bleibenden Teil des Moleküls einen Ether bildet, und Ester und Ether davon können für die Zubereitung eines Medikaments für die Regulierung der Meiose verwendet werden.

3. Die Verwendung gemäß des vorhergehenden Anspruches, worin die Verbindung irgendeine der folgenden Verbindung ist: 7-Oxo-5α-cholest-8-en-3β-ol; 7-Oxo-5α-cholesta-8, 14-dien-3β-ol; 7α-Methyl-5α-cholest-8-en-3β, 7β-diol; 11-Oxo-5α-cholest-8-en-3β-ol; 3β-Hydroxy-5α-cholest-8-en-7-oxim; 3β-Acetoxy-7-oxo-5α-cholest-8-en; 3β-Acetoxy-7-oxo-5α-cholesta-8,14-dien; 7-Oxo-5α-cholest-8-en-3β-yl Benzoat; 7-Methylen-5α-cholest-9-en-3β-ol; 7-Methyl-5α-cholesta-5,8-dien-3β-ol; 11-Oxo-5α-cholest-8-en-3β-yl-Benzoat; Cholesta-8,14-dien-5α-H-3-on; 3α-Fluotcholesta-8,14-dien; Cholesta-2,8,14-trien; und Cholesta-8,14-dien-5α(H)-3-(E), (Z)-oxim.

4. Ein Verfahren zur Regulierung der Meiose in einer Säugetierkeimzelle, welches Verfahren das *ex vivo* Applizieren einer wirksamen Menge einer Verbindung der Formel (I), angegeben in Anspruch 2, in eine Keimzelle, die einer solchen Behandlung bedarf, umfasst, vorzugsweise eine Verbindung, angegeben in Anspruch 3.

5. Ein Verfahren gemäß dem vorhergehenden Anspruch, worin die Keimzelle, deren Meiose reguliert werden muss, eine Oocyte ist.

6. Ein Verfahren gemäß Anspruch 4, wobei eine Verbindung der Formel (I), angegeben in Anspruch 2, vorzugsweise eine Verbindung, angegeben in Anspruch 3, einer Oocyte ex vivo appliziert wird.

7. Ein Verfahren gemäß Anspruch 4, wobei die Keimzelle, deren Meiose reguliert werden soll, eine männliche Keimzelle ist

8. Ein Verfahren gemäß Anspruch 4, wobei männliche Keimzellen durch Applizieren einer Verbindung der Formel (I), angegeben in Anspruch 2, vorzugsweise einer Verbindung angegeben in Anspruch 3, in testikuläres Gewebe *in vitro* hergestellt werden.

## Revendications

1. Utilisation de l'un des composés suivants : 7-oxo-5α-cholesta-8,14-dién-3β-ol ; 7α-méthyl-5α-cholest-8-ène-3β,7β-diol ; 3β-hydroxy-5α-cholest-8-én-7-oxime ; 3β-acétoxy-7-oxo-5α-cholest-8-ène ; 3β-acétoxy-7-oxo-5α-choleata-8,14-diène ; 7-méthylène-5α-cholest-9-én-3β-ol ; 7-méthyl-5α-cholesta-6,8-dién-3β-ol ; cholesta-8,14-diène-5α-H-3-one ; 3α-fluorocholesta-8,14-diène ; cholesta-2,8,14-triène ; et cholesta-8,14-diène-5α(H)-3-(E),(Z)-oxime, en tant que médicament.

2. Utilisation d'un composé de formule générale (I) : 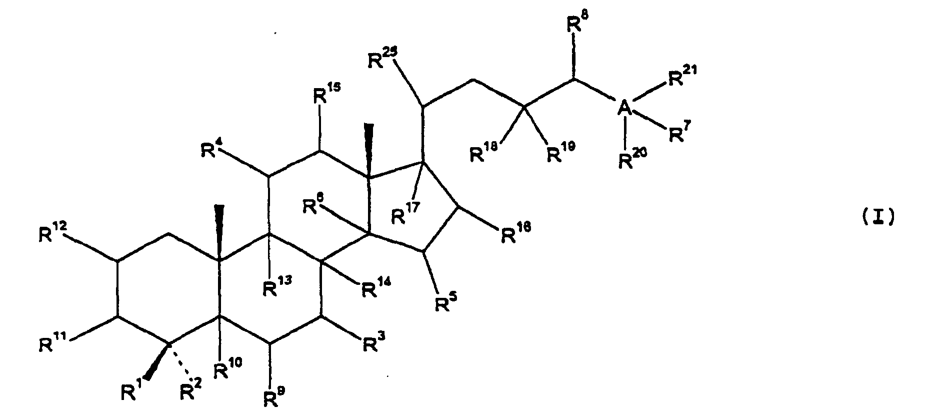 dans laquelle R¹ et R² sont indépendamment choisis dans l'ensemble constitué par l'hydrogène et les radicaux alkyle en C₁ à C₆ ramifiés ou linéaires qui peuvent être substitués par des radicaux halogéno, hydroxy ou cyano, ou bien dans laquelle R¹ et R² désignent ensemble le radical méthylène ou, ensemble avec l'atome de carbone auquel ils sont liés, forment un cycle cyclopropane, un cycle cyclopentane ou un cycle cyclohexane ; R³ est choisi dans l'ensemble constitué par l'hydrogène, un halogène et les radicaux méthylène, hydroxy, méthoxy, acétoxy, oxo, =NOR²⁶ où R²⁶ est l'hydrogène ou un radical alkyle en C₁ à C₃, hydroxy et alkyle en C₁ à C₄ lié au même atome de carbone de la charpente stérol, ou bien R³ désigne, conjointement avec R⁹ ou R¹⁴, une liaison additionnelle entre les atomes de carbone auxquels R³ et R⁹ ou R¹⁴ sont liés ; R⁴ est choisi dans l'ensemble constitué par l'hydrogène, un halogène et les radicaux méthylène, hydroxy, méthoxy, acétoxy, oxo, =NOR²⁷ où R²⁷ est l'hydrogène ou un radical alkyle en C₁ à C₃, hydroxy et alkyle en C₁ à C₄ lié au même atome de carbone de la charpente stérol, ou bien R⁴ désigne, conjointement avec R¹³ ou R¹⁵, une liaison additionnelle entre les atomes de carbone auxquels R⁴ et R¹³ ou R¹⁵ sont liés ; R⁵ est choisi dans l'ensemble constitué par l'hydrogène et les radicaux alkyle en C₁ à C₄, méthylène, hydroxy, méthoxy, oxo et =NOR²² où R²² est l'hydrogène ou un radical alkyle en C₁ à C₃, ou bien R⁵ désigne, conjointement avec R⁶, une liaison additionnelle entre les atomes de carbone auxquels R⁵ et R⁶ sont liés ; R⁶ est l'hydrogène ou bien R⁶ désigne, conjointement avec R⁵, une liaison additionnelle entre les atomes de carbone auxquels R⁵ et R⁶ sont liés ; R⁹ est l'hydrogène ou bien R⁹ désigne, conjointement avec R³ ou R¹⁰, une liaison additionnelle entre les atomes de carbone auxquels R⁹ et R³ ou R¹⁰ sont liés ; R¹⁰ est l'hydrogène ou bien R¹⁰ désigne, conjointement avec R⁹, une liaison additionnelle entre les atomes de carbone auxquels R¹⁰ et R⁹ sont liés ; R¹¹ est choisi dans l'ensemble constitué par les halogènes et les radicaux hydroxy, alcoxy, alcoxy substitué, acyloxy, sulfonyloxy, phosphonyloxy, oxo, -NOR²⁸ où R²⁸ est l'hydrogène ou un radical alkyle en C₁ à C₃, hydroxy et alkyle en C₁ à C₄ lié au même atome de carbone de la charpente stérol, ou bien R¹¹ désigne, conjointement avec R¹², une liaison additionnelle entre les atomes de carbone auxquels R¹¹ et R¹² sont liés ; R¹² est choisi dans l'ensemble constitué par l'hydrogène, les halogènes et les radicaux alkyle en C₁ à C₃, vinyle et alcoxy en C₁ à C₃, ou bien R¹² désigne, conjointement avec R¹¹, une liaison additionnelle entre les atomes de carbone auxquels R¹² et R¹¹ sont liés ; R¹³ est l'hydrogène ou bien R¹³ désigne, conjointement avec R⁴ ou R¹⁴, une liaison additionnelle entre les atomes de carbone auxquels R¹³ et R⁴ ou R¹⁴ sont liés ; R¹⁴ est l'hydrogène ou bien R¹⁴, désigne, conjointement avec R³, R⁶ ou R¹³, une liaison additionnelle entre les atomes de carbone auxquels R¹⁴ et R³ ou R⁶ ou R¹³ sont liés ; R¹⁵ est choisi dans l'ensemble constitué par l'hydrogène et les radicaux alkyle en C₁ à C₄, méthylène, hydroxy, méthoxy, acétoxy, oxo et =NOR²³ où R²³ est l'hydrogène ou un radical alkyle en C₁ à C₃, ou bien R¹⁵ désigne, conjointement avec R⁴, une liaison additionnelle entre les atomes de carbone auxquels R¹⁵ et R⁴ sont liés ; R¹⁶ est choisi dans l'ensemble constitué par l'hydrogène et les radicaux alkyle en C₁ à C₃, méthylène, hydroxy, méthoxy, oxo et =NOR²⁴ où R²⁴ est l'hydrogène ou un radical alkyle en C₁ à C₃, ou bien R¹⁶ désigne, conjointement avec R¹⁷, une liaison additionnelle entre les atomes de carbone auxquels R¹⁶ et R¹⁷ sont liés ; R¹⁷ est l'hydrogène ou le radical hydroxy ou bien R¹⁷ désigne, conjointement avec R¹⁶, une liaison additionnelle entre les atomes de carbone auxquels R¹⁷ et R¹⁶ sont liés ; R¹⁸ et R¹⁹ sont indépendamment l'hydrogène ou le fluor ; R²⁵ est choisi dans l'ensemble constitué par l'hydrogène et les radicaux alkyle en C₁ à C₄, méthylène, hydroxy et oxo ; A est un atome de carbone ou un atome d'azote ; quand A est un atome de carbone, R⁷ est choisi dans l'ensemble constitué par l'hydrogène, le fluor et le radical hydroxy, et R⁸ est choisi dans l'ensemble constitué par l'hydrogène, les halogènes et les radicaux alkyle en C₁ à C₄ et méthylène, ou bien R⁷ désigne, conjointement avec R⁸, une liaison additionnelle entre les atomes de carbone auxquels R⁷ et R⁸ sont liés ; R²⁰ est choisi dans l'ensemble constitué par les radicaux alkyle en C₁ à C₄, trifluorométhyle et cycloalkyle en C₃ à C₆, et R²¹ est choisi dans l'ensemble constitué par les radicaux alkyle en C₁ à C₄, hydroxyalkyle en C₁ à C₄, halogénoalkyle en C₁ à C₄ contenant jusqu'à trois atomes d'halogène, méthoxyméthyle, acétoxyméthyle, et cycloalkyle en C₃ à C₆, ou bien R²⁰ et R²¹, conjointement avec l'atome de carbone auquel ils sont liés, forment un cycle cycloalkyle en C₃ à C₆ ; et quand A est un atome d'azote, R⁷ désigne une paire isolée d'électrons et R⁸ est choisi dans l'ensemble constitué par l'hydrogène et les radicaux alkyle en C₁ à C₄ et oxo ; R²⁰ et R²¹ sont indépendamment des radicaux alkyle en C₁ à C₄ ou cycloalkyle en C₃ à C₆ ; à la condition que le composé de formule générale (I) n'ait aucune double liaison cumulée, et à la condition en outre que le composé ne soit pas un composé de formule générale (II) : 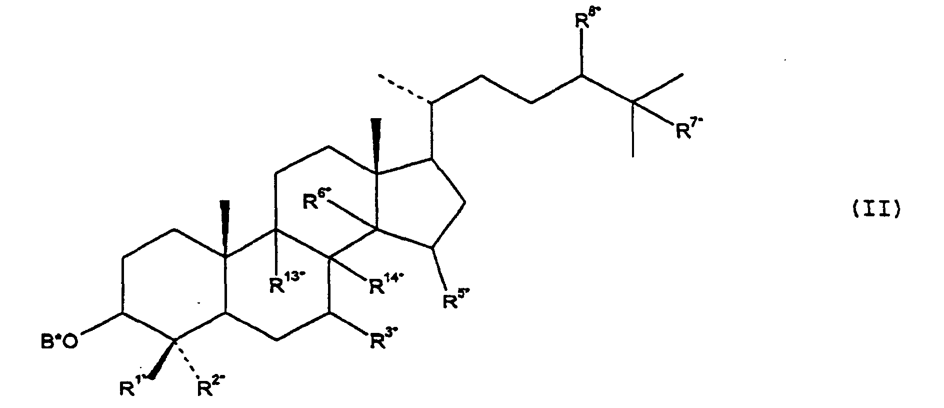 dans laquelle R^{1*} et R²* sont indépendamment choisis dans l'ensemble constitué par l'hydrogène et les radicaux alkyle en C₁ à C₆ ramifiés ou linéaires qui peuvent être substitués par des radicaux halogéno, hydroxy ou cyano, ou bien dans laquelle R^{1*} et R^{2*}, conjointement avec l'atome de carbone auquel ils sont liés, forment un cycle cyclopentane ou un cycle cyclohexane ; R^{13*} et R^{14*} désignent ensemble une liaison additionnelle entre les atomes de carbone auxquels ils sont liés, auquel cas R^{3*} est l'hydrogène et R^{6*} et R^{5*} sont des hydrogènes ou désignent ensemble une liaison additionnelle entre les atomes de carbone auxquels ils sont liés ; ou bien R³* et R⁴* désignent conjointement une liaison additionnelle entre les atomes de carbone auxquels ils sont liés, auquel cas R¹³* est l' hydrogène et R⁶* et R⁵* sont des hydrogènes ou désignent ensemble une liaison additionnelle entre les atomes de carbone auxquels ils sont liés ; ou bien R⁶* et R¹⁴* désignent ensemble une liaison additionnelle entre les atomes de carbone auxquels ils sont liés, auquel cas R¹³*, R³* et R⁵* sont tous des hydrogènes ; R⁸* et R⁷* sont des hydrogènes ou désignent ensemble une liaison additionnelle entre les atomes de carbone auxquels ils sont liés ; et B* est l'hydrogène ou un groupe acyle, y compris un groupe sulfonyle ou un groupe phosphonyle, ou un groupe qui, conjointement avec la partie restante de la molécule, forme un éther,
ainsi que les esters et éthers de ce composé,
pour la préparation d'un médicament destiné à être utilisé dans la régulation de la méiose.

3. Utilisation selon la revendication précédente, dans laquelle le composé est l'un quelconque des composés suivants : 7-oxo-5α-cholest-8-én-3β-ol ; 7-oxo-5α-cholesta-8,14-dién-3β-ol ; 7α-méthyl-5α-cholest-8-ène-3β,7β-diol ; 11-oxo-5α-cholest-8-én-3β-ol ; 3β-hydroxy-5α-cholest-8-én-7-oxime ; 3β-acétoxy-7-oxo-5α-cholest-8-ène ; 3β-acétoxy-7-oxo-5α-cholesta-8,14-diène ; benzoate de 7-oxo-5α-cholest-8-én-3β-yle ; 7-méthylène-5α-cholest-9-én-3β-ol ; 7-méthyl-5α-cholesta-6,8-dién-3β-ol ; benzoate de 11-oxo-5α-cholest-8-én-3β-yle ; cholesta-8,14-diène-5α-H-3-one ; 3α-fluorocholesta-8,14-diène ; cholesta-2,8,14-triène ; et cholesta-8,14-diène-5α(H)-3-(E),(Z)-oxime.

4. Procédé pour réguler la méiose dans une cellule germinale de mammifère, lequel procédé comprend l'administration ex vi vo d'une quantité efficace d'un composé de formule (I) indiqué dans la revendication 2, de préférence d'un composé indiqué dans la revendication 3, à une cellule germinale nécessitant un tel traitement.

5. Procédé selon la revendication précédente, dans lequel la cellule germinale dont la méiose doit être régulée est un oocyte.

6. Procédé selon la revendication 4, dans lequel un composé de formule (I) indiqué dans la revendication 2, de préférence un composé indiqué dans la revendication 3, est administré à un oocyte *ex vivo*.

7. Procédé selon la revendication 4, dans lequel la cellule germinale dont la méiose doit être régulée est une cellule germinale mâle.

8. Procédé selon la revendication 4, grâce auquel des cellules germinales mâles matures sont produites par administration d'un composé de formule (I) indiqué dans la revendication 2, de préférence d'un composé indiqué dans la revendication 3, à du tissu testiculaire *in vitro*.
